# FASCICULE DE BREVET EUROPEEN

(11) **EP 1 013 267 B1**
(45) Date de publication et mention de la délivrance du brevet: **08.08.2001**
(21) Numéro de dépôt: 99402659.9
(22) Date de dépôt: 26.10.1999
(51) Int. Cl.: A61K 7/50, A61K 7/48

(54) **Composition sous forme d'émulsion H/E à forte teneur en cire et ses utilisations dans les domaines cosmétiques et dermatologiques**
Ö/W Emulsionen mit eimen hohen Wachsgehalt und ihre kosmetische und dermatologische Verwendung
O/W emulsions with a high wax content and its uses in cosmetics and dermatology

(30) Priorité: 14.12.1998 FR 9815763
(43) Date de publication de la demande: 28.06.2000
(73) Titulaire: L'OREAL, 75008 Paris (FR)
(72) Inventeur: Burnier, Véronique, 75011 Paris (FR); Roulier, Véronique, 75010 Paris (FR)
(74) Mandataire: Rasson, Catherine

(56) Documents cités:
- EP-A- 0 262 681
- EP-A- 0 667 146
- EP-A- 0 705 593
- EP-A- 0 815 846
- EP-A- 0 835 651
- WO-A-95/15741
- DE-A- 4 337 041
- US-A- 4 233 295
- US-A- 5 665 687
- RIEGER, MARTIN M. AND RHEIN LINDA D.: "Surfactants in Cosmetics" 1997 , MARCEL DEKKER , NEW YORK XP002133261 022912 * page 129 *

## Description

La présente invention se rapporte à une composition sous forme d'une émulsion huile-dans-eau (H/E) comportant une forte teneur en cire, et à ses utilisations dans les domaines cosmétique et dermatologique, notamment pour le soin, le traitement et/ou le maquillage de la peau et/ou des muqueuses, et plus particulièrement pour le traitement des rides et/ou ridules de la peau et/ou pour le traitement et/ou la protection de la peau sèche.

L'invention concerne également un procédé de préparation de cette composition selon lequel on effectue au moins une étape du procédé à l'aide d'un mélangeur-extrudeur.

Il est connu d'utiliser des cires dans des crèmes cosmétiques se présentant sous forme d'émulsions et destinées au soin de la peau humaine, notamment pour les effets antirides apportés par ces cires. Néanmoins, il est difficile d'incorporer dans ces compositions un fort pourcentage de cires car les cires ont tendance à épaissir énormément les émulsions. En outre, quand on incorpore un fort pourcentage de cires dans une émulsion, celle-ci s'applique très difficilement sur la peau car elle ne glisse pas. De plus, il apparaît un effet rêche sur la peau. L'utilisation d'une telle émulsion est donc rédhibitoire pour l'utilisateur.

De plus, il est connu d'incorporer un fort pourcentage de cires dans des mascaras. Ainsi, le document WO-A-95/15741 décrit des mascaras à base de dispersions de cire. Toutefois, ce type de compositions n'est pas utilisable comme produit de soin du fait des inconvénients précédemment cités.

Par ailleurs, pour préparer une émulsion contenant des cires, il est nécessaire de faire fondre les cires dans la phase grasse de l'émulsion, notamment si l'on veut par exemple utiliser des cires telles que les cires de camauba qui sont particulièrement intéressantes pour leur effet antirides sur la peau. Il faut donc chauffer la phase grasse jusqu'à 80-85°C, ce qui est particulièrement préjudiciable lorsque l'on souhaite introduire des composés thermosensibles. Ainsi, le document US-A-4,233,295 décrit des crèmes contenant des cires comme agents gélifiants.

Il subsiste donc le besoin d'une composition contenant un pourcentage important de cires sans les inconvénients de l'art antérieur.

La demanderesse a trouvé de manière surprenante que l'on pouvait incorporer un fort pourcentage de cires dans des émulsions H/E tout en conservant une fluidité satisfaisante et une sensation agréable lors de l'application sur la peau, en préparant l'émulsion à froid à l'aide d'un mélange d'émulsionnants non ioniques, liquide à la température ambiante et ayant un HLB approprié (HLB = Hydrophilic Lipophilic Balance ou balance hydrophile lipophile) et à partir d'une phase huileuse souple contenant un fort pourcentage de cires.

La présente invention a donc pour objet une composition sous forme d'une émulsion huile-dans-eau comportant une phase huileuse dispersée dans une phase aqueuse, caractérisée en ce qu'elle contient un mélange d'au moins deux émulsionnants non ioniques, ledit mélange étant liquide à la température ambiante et ayant un HLB allant de 6 à 13, et en ce que la phase huileuse contient une huile et au moins 5 % en poids d'une ou plusieurs cires par rapport au poids total de la composition, au moins une cire étant choisie parmi les cires ayant une température de fusion commençante supérieure ou égale à 50°C.

La composition obtenue bien que contenant un fort taux de cire est fraîche à l'application.

La demanderesse a cherché à éviter l'utilisation d'émulsionnants ioniques qui peuvent être irritants pour certains types de peau et notamment les peaux sensibles. Toutefois, l'utilisation d'émulsionnants non ioniques pose des difficultés dans la mesure où il n'existe pas d'émulsionnant non ionique liquide à la température ambiante et ayant un HLB allant de 6 à 13, permettant d'atteindre le but de l'invention. La demanderesse a donc été amenée à utiliser un mélange d'émulsionnants non ioniques, répondant aux critères souhaités.

Le mélange d'émulsionnants non ioniques, utilisé dans la composition de l'invention, doit être liquide à la température ambiante, c'est-à-dire à une température allant de 15°C à 25°C, et avoir un HLB allant de 6 à 13. Il peut comprendre notamment (1) au moins un émulsionnant non ionique ayant un HLB égal ou supérieur à 13 et (2) au moins un émulsionnant non ionique ayant un HLB égal ou inférieur à 5. En outre, il peut comprendre éventuellement au moins un co-émulsionnant qui peut être notamment nécessaire si les émulsionnants (1) et (2) utilisés sont tous deux solides, le co-émulsionnant étant alors liquide et permettant d'obtenir un mélange liquide.

De manière connue, le HLB d'un mélange d'émulsionnants correspond à la moyenne des HLB de chacun des émulsionnants constituant le mélange, compte tenu de la proportion pondérale de ces émulsionnants.

Comme émulsionnants non ioniques de HLB égal ou inférieur à 5, on peut citer par exemple les esters de polyols et d'acide gras ayant une chaîne alkyle comportant de 12 à 22 atomes de carbone, tels que les esters d'acide gras et de glycérine, de glucose ou de sorbitol ; les dérivés oxyéthylénés des esters de polyols et d'acide gras ayant une chaîne alkyle comportant de 12 à 22 atomes de carbone, comportant de 1 à 50 groupes oxyéthylénés, tels que le complexe de triisostéarine (triester de glycérine et d'acide isostéarique) et de PEG-6 ; les éthers de polyéthylène glycol et d'alcool gras ayant une chaîne alkyle comportant de 12 à 22 atomes de carbone, comportant de 1 à 50 groupes oxyéthylénés, tels que les éthers d'oléyle et en particulier l'oleth-25 (25 groupes oxyéthylénés), et leurs mélanges.

Comme émulsionnants non ioniques de HLB égal ou inférieur à 5, on peut utiliser avantageusement ceux qui sont liquides à température ambiante, tels que les esters gras et éthers gras de polyol, à chaîne ramifiée ou insaturée comportant de 12 à 22 atomes de carbone, et en particulier le mono-isostéarate de sorbitan comme le produit vendu sous la dénomination "Arlacel 987" par la société ICI, le mono/di-oléate de sorbitan comme le produit vendu sous la dénomination "Arlacel 83" par la société ICI, le complexe de triisostéarine et de PEG-6 comme le produit vendu sous la dénomination "Labrafil isostearique" par la société Gattefossé, le penta-isostéarate de décaglycéryle comme le produit vendu sous la dénomination "Nikkol Decaglyn 5-IS" par la société Nikko Chemical, le dioléate de méthylglucose comme le produit vendu sous la dénomination "Isolan DO" par la société Goldschmidt.

Comme émulsionnants non ioniques de HLB égal ou supérieur à 13, on peut citer par exemple les esters de polyéthylène glycol et d'acide gras ayant une chaîne alkyle comportant de 12 à 22 atomes de carbone, comportant de 5 à 100 et de préférence de 20 à 60 groupes oxyéthylénés, tels que le stéarate de PEG-40 ; les éthers de polyéthylène glycol et d'alcool gras ayant une chaîne alkyle comportant de 12 à 22 atomes de carbone, comportant de 5 à 100 et de préférence de 10 à 30 groupes oxyéthylénés, tels que le ceteareth-25 ou le ceteth-25 ; les esters de sorbitan et d'acide gras ayant une chaîne alkyle comportant de 12 à 22 atomes de carbone, comportant de 0 à 100 et de préférence de 4 à 25 groupes oxyéthylénés, tels que le Polysorbate 20, le Polysorbate 40 et lé Polysorbate 60 ; les esters de sucre et d'acide gras ayant une chaîne alkyle comportant de 12 à 22 atomes de carbone, tels que le stéarate de sucrose ; les dérivés de polyéthylène glycol et d'esters de glycérine et d'acide gras ayant une chaîne alkyle comportant de 12 à 22 atomes de carbone, tels que le PEG-8 caprylic/capric glycerides ; les éthers de polyéthylène glycol des esters de méthyl glucose et d'acide gras ayant une chaîne alkyle comportant de 12 à 22 atomes de carbone, tels que le PEG-20 methyl glucose sesquistéarate ; et leurs mélanges.

Comme émulsionnants non ioniques de HLB égal ou supérieur à 13, on peut utiliser avantageusement ceux qui sont liquides à température ambiante, tels que le Polysorbate 20 comme le produit vendu sous la dénomination "Tween 20" par la société ICI, le Polysorbate 40 comme le produit vendu sous la dénomination "Tween 40" par la société ICI, le PEG-8 caprylic/capric glycerides comme le produit vendu sous la dénomination "Labrasol" par la société Gattefossé, le PEG-20 methyl glucose sesquistéarate comme le produit vendu sous la dénomination "Glucamate SSE 20" par la société Amerchol.

Comme co-émulsionnant, on peut citer par exemple les alcools gras comportant une chaîne ramifiée ou insaturée ayant de 8 à 22 atomes de carbone, tels que l'alcool isostéarique ; les acides gras comportant une chaîne ramifiée ou insaturée ayant de 8 à 22 atomes de carbone, tels que l'acide ricinoléique ; les esters de polyol et d'acide gras ramifié comportant de 8 à 22 atomes de carbone, tels que les esters gras ramifiés de glycéryle ou de propylène glycol comme l'isostéarate de glycéryle ou l'isostéarate de propylène glycol, et leurs mélanges.

Comme mélange d'émulsionnants non ioniques, liquide à température ambiante et ayant un HLB de 6 à 13, utilisable dans la composition selon l'invention, on peut citer par exemple le mélange de stéarate de glycéryle, stéarate de propylène glycol, isostéarate de glycéryle, isostéarate de propylène glycol, oleth-25 et ceteth-25, commercialisé par la société Gattefosse sous la dénomination "Hydrolactol 70" de HLB 10.

La quantité de phase huileuse dans la composition de l'invention va généralement de 10 à 70 % et de préférence de 20 à 50 % en poids par rapport au poids total de la composition. Cette phase huileuse est utilisée en une quantité telle ou bien contient une quantité de cires telle que la quantité de cires dans la composition finale est égale ou supérieure à 5 %.

La phase huileuse de la composition de l'invention comprend une ou plusieurs huiles choisies parmi les huiles d'origine animale, les huiles d'origine végétale, les huiles minérales, les huiles synthétiques, les huiles fluorées, les huiles de silicone et notamment les huiles de silicone volatiles. Elle comprend généralement un ou plusieurs corps gras choisis parmi les gommes de silicone, les résines de silicone, les alcools gras, les acides gras et les élastomères de silicone tels que les produits commercialisés sous la dénomination "KSG" par la société Shin-Etsu, sous la dénomination "Trefil" par la société Dow Corning du sous la dénomination "Gransil" par la société General Electric.

De manière avantageuse, la composition de l'invention peut aussi contenir une ou plusieurs charges (constituants pulvérulents) qui peuvent être choisies par exemple dans le groupe formé par le talc ; les micas d'origine naturelle ou synthétique ; le kaolin ; les oxydes de zinc ou de titane ; le carbonate de calcium ; le carbonate et l'hydrocarbonate de magnésium ; la silice, en particulier la silice sphérique, la poudre de silice commercialisée sous la dénomination "Cab-O-Sil TS 530" par la société Cabot, et les microbilles de silice telles que celles commercialisées sous la dénomination SB150 par la société Myoshi ; le dioxyde de titane ; les billes de verre et de céramique commercialisées par la société 3M sous la dénomination commerciale "Macrolite" ; les savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium ; les poudres de polymères synthétiques non expansées, telles que les poudres de polyéthylène, de polystyrène, de polyesters, de polyamides (par exemple te nylon ou la poly-β-alanine), de copolymères d'acrylates (par exemple les microsphères microporeuses vendues par la société Dow Corning sous la dénomination commerciale "Polytrap"), d'acides polyméthacryliques, de polystyrène, de téflon comme le "Fluon" ; les poudres expansées telles que les microsphères creuses en matériau thermoplastique préparées par des procédés connus, comme ceux décrits dans US-A-3 615 972 et EP-A-0 56219 et notamment les microsphères commercialisées sous la dénomination commerciale "Expancel" par la société Kemanord Plast ou sous la dénomination commerciale "Micropearl F 80 ED" par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination "Dry-Flo" par la société National Starch ; les microbilles de résine de silicone telles que celles commercialisées sous la dénomination "Tospearl" par la société Toshiba Silicone, et leurs mélanges.

La quantité de phase huileuse dans la composition de l'invention va généralement de 10 à 70 % et de préférence de 20 à 50 % en poids par rapport au poids total de la composition. Cette phase huileuse est utilisée en une quantité telle ou bien contient une quantité de cires telle que la quantité de cires dans la composition finale est égale ou supérieure à 5 %.

La phase huileuse de la composition de l'invention comprend d'une huile choisis parmi les huiles d'origine animale, les huiles d'origine végétale, les huiles minérales, les huiles synthétiques, les huiles fluorées, les huiles de silicone et notamment les huiles de silicone volatiles, les gommes de silicone, les résines de silicone, les alcools gras, les acides gras et les élastomères de silicone tels que les produits commercialisés sous la dénomination "KSG" par la société Shin-Etsu, sous la dénomination "Trefil" par la société Dow Corning ou sous la dénomination "Gransil" par la société General Electric.

De manière avantageuse, la composition de l'invention peut aussi contenir une ou plusieurs charges (constituants pulvérulents) qui peuvent être choisies par exemple dans le groupe formé par le talc ; les micas d'origine naturelle ou synthétique ; le kaolin ; les oxydes de zinc ou de titane ; le carbonate de calcium ; le carbonate et l'hydrocarbonate de magnésium ; la silice, en particulier la silice sphérique, la poudre de silice commercialisée sous la dénomination "Cab-O-Sil TS 530" par la société Cabot, et les microbilles de silice telles que celles commercialisées sous la dénomination SB150 par la société Myoshi ; le dioxyde de titane ; les billes de verre et de céramique commercialisées par la société 3M sous la dénomination commerciale "Macrolite" ; les savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium ; les poudres de polymères synthétiques non expansées, telles que les poudres de polyéthylène, de polystyrène, de polyesters, de polyamides (par exemple le nylon ou la poly-ß-alanine), de copolymères d'acrylates (par exemple les microsphères microporeuses vendues par la société Dow Corning sous la dénomination commerciale "Polytrap"), d'acides polyméthacryliques, de polystyrène, de téflon comme le "Fluon" ; les poudres expansées telles que les microsphères creuses en matériau thermoplastique préparées par des procédés connus, comme ceux décrits dans US-A-3 615 972 et EP-A-0 56219 et notamment les microsphères commercialisées sous la dénomination commerciale "Expancel" par la société Kemanord Plast ou sous la dénomination commerciale "Micropearl F 80 ED" par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination "Dry-Flo" par la société National Starch ; les microbilles de résine de silicone telles que celles commercialisées sous la dénomination "Tospearl" par la société Toshiba Silicone, et leurs mélanges.

Les charges peuvent représenter jusqu'à 20 % en poids par rapport au poids total de la composition, et de préférence de 1 à 12 % en poids par rapport au poids total de la composition.

La phase aqueuse de la composition de l'invention représente au moins 30 % en poids par rapport au poids total de la composition et de préférence de 50 à 80 % en poids par rapport au poids total de la composition.

La composition selon l'invention peut être utilisée dans tous les domaines où ce type de forme galénique est intéressant, et notamment dans les domaines cosmétique et dermatologique. Quand elle constitue une composition cosmétique et/ou dermatologique, elle contient avantageusement un milieu physiologiquement acceptable, c'est-à-dire compatible avec la peau, les muqueuses, les ongles et/ou les cheveux.

Les compositions, objet de l'invention, trouvent leur application dans un grand nombre de traitements de la peau, des muqueuses (lèvres) et des cheveux, y compris le cuir chevelu, notamment pour la protection, le soin, le nettoyage et/ou le maquillage de la peau et/ou des muqueuses, pour la protection, le soin et/ou le nettoyage des cheveux et/ou pour le traitement thérapeutique de la peau, des cheveux et/ou des muqueuses.

Les compositions selon l'invention peuvent par exemple être utilisées comme produits de traitement, de soin, de protection et/ou de nettoyage de la peau sous forme de crèmes ou de laits, ou comme produits de maquillage (peau et lèvres) par incorporation de charges et/ou de matières colorantes (pigments et/ou colorants). Elles sont particulièrement appropriées pour le traitement des rides et/ou ridules de la peau et pour le traitement et/ou la protection de la peau sèche.

Aussi, l'invention a encore pour objet l'utilisation cosmétique non-thérapeutique de la composition telle que définie ci-dessus pour le traitement, la protection, le soin et/ou le nettoyage de la peau, des muqueuses et/ou les cheveux, et/ou pour le maquillage de la peau et/ou des muqueuses.

Elle a aussi pour objet l'utilisation cosmétique non-thérapeutique de la composition telle que définie ci-dessus pour le traitement des rides et/ou des ridules de la peau.

L'invention a encore pour objet l'utilisation de la composition telle que définie ci-dessus pour la fabrication d'une composition dermatologique destinée au traitement et/ou à la protection de la peau sèche.

En outre, de façon connue, les compositions de l'invention peuvent contenir des adjuvants habituels dans le domaine cosmétique ou dermatologique, tels que des actifs hydrophiles ou lipophiles, des conservateurs, des antioxydants, des parfums, des solvants, des filtres solaires, des matières colorantes, des agents basiques ou acides et encore des vésicules lipidiques. Ces adjuvants sont utilisés dans les proportions habituelles dans le domaine cosmétique ou dermatologique, et par exemple de 0,01 à 30 % du poids total de la composition, et ils sont, selon leur nature, introduits dans la phase aqueuse ou dans la phase huileuse de la composition, ou encore dans des vésicules. Ces adjuvants ainsi que leurs concentrations doivent être tels qu'ils ne modifient pas la propriété recherchée pour la composition.

Si l'on souhaite obtenir une composition moins fluide et/ou améliorer la stabilité de l'émulsion, on peut y ajouter un ou plusieurs gélifiants hydrophiles tels que les polymères carboxyvinyliques ou carbomers et les polyacrylamides. Selon un mode préféré de réalisation de l'invention, on utilise un polyacrylamide tel que le produit commercialisé sous la dénomination Hostacerin AMPS par la société Hoechst. Ces gélifiants sont utilisés à des concentrations allant de 0,05 à 2 %, de préférence 0,1 à 0,5 % en poids par rapport au poids total de la composition.

Comme actifs utilisables dans la composition de l'invention, on peut citer par exemple les hydratants tels que les polyols et notamment la glycérine, l'éthylène glycol, l'isoprène-glycol, le propane-1,2 diol, la diglycérine, le sorbitol, les polyéthylène glycols et leurs mélanges.

La composition selon l'invention peut être préparée de manière avantageuse en utilisant, pour au moins une étape du procédé, un appareil de malaxage tel qu'un broyeur à cylindres comportant deux cylindres tournant en sens inverse entre lesquels passe la pâte ou un mélangeur-extrudeur à vis. On utilise de préférence un mélangeur-extrudeur à vis.

Un autre objet de l'invention est donc un procédé de préparation d'une composition selon l'invention, caractérisé en ce que l'on effectue au moins une étape du procédé à l'aide d'un mélangeur-extrudeur à vis.

Selon un premier mode de réalisation de l'invention, le procédé de préparation comprend les étapes suivantes :
- (1) préparation de la phase huileuse sous forme d'une pâte souple obtenue en faisant un prémélange des cires et huiles, en chauffant ce prémélange à une température laquelle il fond, puis en introduisant dans un mélangeur-extrudeur à vis soumis à un gradient de température allant de 80°C à 20°C, en une ou plusieurs fois, le prémélange fondu et les autres constituants (notamment les charges) de la phase huileuse, et en malaxant le mélange obtenu tout en le refroidissant jusqu'à température ambiante tandis qu'il est amené à la sortie du mélangeur-extrudeur ;
- (2) incorporation du mélange d'émulsionnants (émulsionnants et co-émulsionnants) dans la pâte souple obtenue en (1), et
- (3) incorporation, sous agitation, du mélange obtenu en (2) dans la phase aqueuse.

Dans ce mode de réalisation, les étapes (2) et (3) sont réalisées dans un appareil de mélange habituellement utilisé par l'homme du métier, tel qu'un rotor stator.

En outre, dans le procédé décrit ci-dessus, l'émulsionnant utilisé est un mélange d'émulsionnants non ioniques, liquide à température ambiante et ayant un HLB de 6 à 13, et les quantités utilisées sont telles que l'émulsion obtenue comporte une huile et au moins 5 % de cire en poids par rapport au poids total de la composition.

Comme indiqué plus haut, le mélange des phases huileuse et aqueuse se faisant à froid, l'incorporation de composés thermosensibles ne pose pas de problème.

Selon un mode particulier de réalisation de l'invention, les étapes (2) et (3) ci-dessus sont également réalisées dans le mélangeur-extrudeur à vis utilisé pour l'étape (1). Le mélange d'émulsionnants et la phase aqueuse sont alors introduits dans une partie (ou élément) du mélangeur-extrudeur où la température est proche de la température ambiante.

L'utilisation d'un mélangeur-extrudeur permet d'obtenir une pâte de phase huileuse de qualité très constante de façon reproductible. De plus, il est possible, er adaptant la filière de sortie du mélangeur-extrudeur, de conditionner la composition en ligne à la sortie dudit mélangeur-extrudeur.

Les différents étapes du procédé peuvent être réalisées dans un ou plusieurs extrudeurs disposés à la suite les uns des autres, et de préférence dans un extrudeur bivis unique.

Les conditions dans lesquelles l'extrusion peut être effectuée sont décrites dans le document FR-A-2,715,306 dont le contenu est incorporé à la présente demande par référence.

L'invention est illustrée plus en détail dans l'exemple suivant. Les quantités indiquées sont des pourcentages en poids.

### Exemple : crème de soin

### Phase huileuse

- Dry-Flo (charge) 15 %
- Cire microcristalline 19 %
- Huile minérale qsp 100 %

### Emulsion H/E

- Phase huileuse 30 %
- Hydrolactol 70 (mélange d'émulsionnants) 20 %
- Ammonium polyacryldimethyltauramide (Hostacerin AMPS) 0,1 %
- Eau qsp 100 %

### Mode opératoire 1 :

- On chauffe le mélange de cire et d'huile à environ 100°C,
- on introduit le mélange fondu dans un mélangeur-extrudeur en même temps que la charge, on obtient à la sortie du mélangeur-extrudeur la phase huileuse sous forme d'une pâte souple,
- Dans un rotor stator, on incorpore dans la pâte souple le mélange d'émulsionnants,
- puis on ajoute peu à peu le mélange obtenu dans la phase aqueuse (eau et Hostacerin AMPS) tout en agitant.

### Mode opératoire 2:

- On chauffe le mélange de cire et d'huile à environ 100°C,
- on introduit la charge dans l'élément de tête d'un mélangeur-extrudeur comportant au moins six éléments,
- on introduit la phase huileuse dans le second élément dudit mélangeur-extrudeur à vis, et
- introduit la phase aqueuse et le mélange d'émulsionnants par deux entrées différentes, dans le quatrième élément dudit mélangeur-extrudeur à vis.

Les éléments du mélangeur-extrudeur à vis utilisé sont, en allant du premier au sixième élément, portés respectivement aux températures suivantes : 20°C, 80°C, 60°C, 20°C, 20°C et 20°C.

On obtient une crème qui présente une texture très légère et qui possède de bonnes qualités d'hydratation et est apte à lisser le relief de la peau.

## Revendications

1. Composition sous forme d'une émulsion huile-dans-eau comportant une phase huileuse dispersée dans une phase aqueuse, caractérisée en ce qu'elle contient un mélange d'au moins deux émulsionnants non ioniques, ledit mélange étant liquide à la température ambiante et ayant un HLB allant de 6 à 13, et en ce que la phase huileuse contient une huile et au moins 5 % en poids d'une ou plusieurs cires par rapport au poids total de la composition, au moins une cire étant choisie parmi les cires ayant une température de fusion commençante supérieure ou égale à 50°C.

2. Composition selon la revendication 1, caractérisée en ce que le mélange d'émulsionnants comprend (1) au moins un émulsionnant non ionique ayant un HLB égal ou supérieur à 13 et (2) au moins un émulsionnant non ionique ayant un HLB égal ou inférieur à 5.

3. Composition selon la revendication 1 ou 2, caractérisée en ce que le mélange d'émulsionnants comprend en outre au moins un co-émulsionnant.

4. Composition selon la revendication 2 ou 3, caractérisée en ce que l'émulsionnant non ionique ayant un HLB égal ou supérieur à 13 est choisi dans le groupe comprenant les esters de polyéthylène glycol et d'acide gras ayant une chaîne alkyle comportant de 12 à 22 atomes de carbone, comportant de 5 à 100 groupes oxyéthylénés ; les éthers de polyéthylène glycol et d'alcool gras ayant une chaîne alkyle comportant de 12 à 22 atomes de carbone, comportant de 5 à 100 groupes oxyéthylénés ; les esters de sorbitan et d'acide gras ayant une chaîne alkyle comportant de 12 à 22 atomes de carbone, comportant de 0 à 100 groupes oxyéthylénés ; les esters de sucre et d'acide gras ayant une chaîne alkyle comportant de 12 à 22 atomes de carbone ; les dérivés de polyéthylène glycol et d'esters de glycérine et d'acide gras ayant une chaîne alkyle comportant de 12 à 22 atomes de carbone ; les éthers de polyéthylène glycol des esters de méthyl glucose et d'acide gras ayant une chaîne alkyle comportant de 12 à 22 atomes de carbone ; et leurs mélanges.

5. Composition selon l'une quelconque des revendications 2 à 4, caractérisée en ce que l'émulsionnant non ionique de HLB égal ou inférieur à 5 est choisi dans le groupe comprenant les esters de polyols et d'acide gras ayant une chaîne alkyle comportant de 12 à 22 atomes de carbone ; les dérivés oxyéthylénés des esters de polyols et d'acide gras ayant une chaîne alkyle comportant de 12 à 22 atomes de carbone, comportant de 1 à 50 groupes oxyéthylénés ; les éthers de polyéthylène glycol et d'alcool gras ayant une chaîne alkyle comportant de 12 à 22 atomes de carbone, comportant de 1 à 50 groupes oxyéthylénés, et leurs mélanges.

6. Composition selon l'une quelconque des revendications 3 à 5, caractérisée en ce que le co-émulsionnant est choisi dans le groupe comprenant les alcools gras comportant une chaîne ramifiée ou insaturée ayant de 8 à 22 atomes de carbone ; les acides gras comportant une chaîne ramifiée ou insaturée ayant de 8 à 22 atomes de carbone ; les esters de polyol et d'acide gras ramifié comportant de 8 à 22 atomes de carbone, et leurs mélanges.

7. Composition selon l'une quelconque des revendications précédentes, caractérisée en ce que le mélange d'émulsionnants comprend du stéarate de glycéryle, du stéarate de propylène glycol, de l'isostéarate de glycéryle, de l'isostéarate de propylène glycol, de l'oleth-25 et du ceteth-25.

8. Composition selon l'une quelconque des revendications précédentes, caractérisée en ce que la quantité de mélange d'émulsionnants va de 0,1 à 30 % en poids en matière active par rapport au poids total de la composition.

9. Composition selon l'une quelconque des revendications précédentes, caractérisée en ce que la cire est choisie dans le groupe comprenant les cires minérales, les cires animales, les cires végétales, les huiles hydrogénées, les esters gras et les glycérides concrets à 25°C, les cires synthétiques, les cires de silicone et leurs mélanges.

10. Composition selon l'une quelconque des revendications précédentes, caractérisée en ce que la cire est choisie parmi la cire de Carnauba, les cires de polyéthylène ayant une température de fusion commençante supérieure à 65°C, les cires microcristallines ayant une température de fusion commençante supérieure à 65°C, et leurs mélanges.

11. Composition selon l'une quelconque des revendications précédentes, caractérisée en ce que la quantité de cire(s) va de 5 à 30 % en poids par rapport au poids total de la composition.

12. Composition selon l'une quelconque des revendications précédentes, caractérisée en ce que la quantité de phase huileuse va de 10 à 70 % en poids par rapport au poids total de la composition.

13. Composition selon l'une quelconque des revendications précédentes, caractérisée en ce que la phase huileuse comprend, en outre, un ou plusieurs corps gras choisis parmi les huiles d'origine animale, les huiles d'origine végétale, les huiles minérales, les huiles synthétiques, les huiles fluorées, les huiles de silicone et notamment les huiles de silicone volatiles, les gommes de silicone, les résines de silicone, les alcools gras, les acides gras et les élastomères de silicone.

14. Composition selon l'une quelconque des revendications précédentes, caractérisée en ce qu'elle comprend en outre au moins une charge.

15. Composition selon la revendication précédente, caractérisée en ce que la charge est choisie dans le groupe formé par le talc, les micas, le kaolin, les oxydes de zinc ou de titane, le carbonate de calcium, le carbonate et l'hydrocarbonate de magnésium, la silice, le dioxyde de titane, les billes de verre et de céramique, les savons métalliques, les poudres de polymères synthétiques non expansées, les poudres expansées, les poudres de matériaux organiques naturels, les microbilles de résine de silicone et leurs mélanges.

16. Composition selon la revendication 14 ou 15, caractérisée en ce que la quantité de charge(s) va de 1 à 12 % en poids par rapport au poids total de la composition.

17. Composition selon l'une quelconque des revendications précédentes, caractérisée en ce que la phase aqueuse représente de 50 à 80 % en poids par rapport au poids total de la composition.

18. Composition selon l'une quelconque des revendications précédentes, caractérisée en ce que la phase aqueuse comprend au moins un gélifiant choisi parmi les polyacrylamides.

19. Composition selon l'une quelconque des revendications précédentes, caractérisée en ce qu'elle constitue une composition cosmétique et/ou dermatologique.

20. Utilisation cosmétique non-thérapeutique de la composition selon l'une quelconque des revendications 1 à 18 pour le traitement, la protection, le soin et/ou le nettoyage de la peau, des muqueuses et/ou les cheveux, et/ou pour le maquillage de la peau et/ou des muqueuses.

21. Utilisation cosmétique non-thérapeutique de la composition selon l'une quelconque des revendications 1 à 18 pour le traitement des rides et/ou des ridules de la peau.

22. Utilisation de la composition selon l'une quelconque des revendications 1 à 18 pour la fabrication d'une composition dermatologique destinée au traitement et/ou à la protection de la peau sèche.

23. Procédé de préparation d'une composition selon l'une quelconque des revendications 1 à 19, caractérisé en ce que l'on effectue au moins une étape du procédé à l'aide d'un mélangeur-extrudeur à vis.

24. Procédé selon la revendication 23, caractérisé en ce qu'il comprend les étapes suivantes:
- (1) préparation de la phase huileuse sous forme d'une pâte souple obtenue en faisant un prémélange des cires et huiles, en chauffant ce prémélange à une température à laquelle il fond, puis en introduisant dans un mélangeur-extrudeur à vis soumis à un gradient de température allant de 80°C à 20°C, en une ou plusieurs fois, le prémélange fondu et les autres constituants de la phase huileuse, et en malaxant le mélange obtenu tout en le refroidissant jusqu'à température ambiante tandis qu'il est amené à la sortie du mélangeur-extrudeur ;
- (2) incorporation du mélange d'émulsionnants dans la pâte souple obtenue en (1), et
- (3) incorporation, sous agitation, du mélange obtenu en (2) dans la phase aqueuse.

25. Procédé selon la revendication 23 ou 24, caractérisé en ce que les étapes (2) et (3) sont réalisées dans le mélangeur-extrudeur à vis de l'étape (1).

26. Procédé selon l'une quelconque des revendications 23 à 25, caractérisé en ce que la phase huileuse se présente sous forme d'une pâte souple ayant une viscosité dynamique à 25°C comprise entre 3 et 35 Pa.s, mesurée avec un viscosimètre rotatif CONTRAVES TV équipé d'un mobile "MS-r4" à la fréquence de 60 Hz.

## Claims

1. Composition in the form of an oil-in-water emulsion comprising an oily phase dispersed in an aqueous phase, characterized in that it comprises a mixture of at least two nonionic emulsifiers, the said mixture being liquid at ambient temperature and having an HLB ranging from 6 to 13, and in that the oily phase comprises an oil and at least 5% by weight of one or more waxes with respect to the total weight of the composition, at least one wax being chosen from waxes having a starting melting temperature of greater than or equal to 50°C.

2. Composition according to Claim 1, characterized in that the mixture of emulsifiers comprises (1) at least one nonionic emulsifier having an HLB equal to or greater than 13 and (2) at least one nonionic emulsifier having an HLB equal to or less than 5.

3. Composition according to Claim 1 or 2, characterized in that the mixture of emulsifiers additionally comprises at least one coemulsifier.

4. Composition according to Claim 2 or 3, characterized in that the nonionic emulsifier having an HLB equal to or greater than 13 is chosen from the group comprising esters of polyethylene glycol and of a fatty acid having an alkyl chain comprising from 12 to 22 carbon atoms, which esters comprise from 5 to 100 oxyethylene groups; ethers of polyethylene glycol and of a fatty alcohol having an alkyl chain comprising from 12 to 22 carbon atoms, which ethers comprise from 5 to 100 oxyethylene groups; esters of sorbitan and of a fatty acid having an alkyl chain comprising from 12 to 22 carbon atoms, which esters comprise from 0 to 100 oxyethylene groups; esters of sugar and of a fatty acid having an alkyl chain comprising from 12 to 22 carbon atoms; derivatives of polyethylene glycol and of esters of glycerol and of a fatty acid having an alkyl chain comprising from 12 to 22 carbon atoms; polyethylene glycol ethers of esters of methyl glucose and of a fatty acid having an alkyl chain comprising from 12 to 22 carbon atoms; and their mixtures.

5. Composition according to any one of Claims 2 to 4, characterized in that the nonionic emulsifier with an HLB equal to or less than 5 is chosen from the group comprising esters of polyols and of fatty acids having an alkyl chain comprising from 12 to 22 carbon atoms; oxyethylenated derivatives of esters of polyols and of a fatty acid having an alkyl chain comprising from 12 to 22 carbon atoms, which derivatives comprise from 1 to 50 oxyethylene groups; ethers of polyethylene glycol and of a fatty alcohol having an alkyl chain comprising from 12 to 22 carbon atoms, which ethers comprise from 1 to 50 oxyethylene groups, and their mixtures.

6. Composition according to any one of Claims 3 to 5, characterized in that the coemulsifier is chosen from the group comprising fatty alcohols comprising a branched or unsaturated chain having from 8 to 22 carbon atoms; fatty acids comprising a branched or unsaturated chain having from 8 to 22 carbon atoms; esters of a polyol and of a branched fatty acid comprising from 8 to 22 carbon atoms, and their mixtures.

7. Composition according to any one of the preceding claims, characterized in that the mixture of emulsifiers comprises glyceryl stearate, propylene glycol stearate, glyceryl isostearate, propylene glycol isostearate, oleth-25 and ceteth-25.

8. Composition according to any one of the preceding claims, characterized in that the amount of mixture of emulsifiers ranges from 0.1 to 30% by weight of active material with respect to the total weight of the composition.

9. Composition according to any one of the preceding claims, characterized in that the wax is chosen from the group comprising mineral waxes, animal waxes, vegetable waxes, hydrogenated oils, fatty esters and glycerides which are solid at 25°C, synthetic waxes, silicone waxes, and their mixtures.

10. Composition according to any one of the preceding claims, characterized in that the wax is chosen from carnauba wax, polyethylene waxes having a starting melting temperature of greater than 65°C, microcrystalline waxes having a starting melting temperature of greater than 65°C, and their mixtures.

11. Composition according to any one of the preceding claims, characterized in that the amount of wax(es) ranges from 5 to 30% by weight with respect to the total weight of the composition.

12. Composition according to any one of the preceding claims, characterized in that the amount of oily phase ranges from 10 to 70% by weight with respect to the total weight of the composition.

13. Composition according to any one of the preceding claims, characterized in that the oily phase additionally comprises one or more fatty substances chosen from oils of animal origin, oils of vegetable origin, mineral oils, synthetic oils, fluorinated oils, silicone oils, in particular volatile silicone oils, silicone gums, silicone resins, fatty alcohols, fatty acids and silicone elastomers.

14. Composition according to any one of the preceding claims, characterized in that it additionally comprises at least one filler.

15. Composition according to the preceding claim, characterized in that the filler is chosen from the group formed by talc, micas, kaolin, zinc or titanium oxides, calcium carbonate, magnesium carbonate and hydrocarbonate, silica, titanium dioxide, glass and ceramic beads, metal soaps, powders formed from non-expanded synthetic polymers, expanded powders, powders formed from natural organic materials, silicone resin microbeads, and their mixtures.

16. Composition according to Claim 14 or 15, characterized in that the amount of filler(s) ranges from 1 to 12% by weight with respect to the total weight of the composition.

17. Composition according to any one of the preceding claims, characterized in that the aqueous phase represents from 50 to 80% by weight with respect to the total weight of the composition.

18. Composition according to any one of the preceding claims, characterized in that the aqueous phase comprises at least one gelling agent chosen from polyacrylamides.

19. Composition according to any one of the preceding claims, characterized in that it constitutes a cosmetic and/or dermatological composition.

20. Non-therapeutic cosmetic use of the composition according to any one of Claims 1 to 18 in treating, protecting, caring for and/or cleansing the skin, mucous membranes and/or hair and/or in making up the skin and/or mucous membranes.

21. Non-therapeutic cosmetic use of the composition according to any one of Claims 1 to 18 in treating wrinkles and/or fine lines of the skin.

22. Use of the composition according to any one of Claims 1 to 18 in the manufacture of a dermatological composition intended for treating and/or protecting dry skin.

23. Process for the preparation of a composition according to any one of Claims 1 to 19, characterized in that at least one stage of the process is carried out using a screw mixer-extruder.

24. Process according to Claim 23, characterized in that it comprises the following stages:
- (1) preparation of the oily phase in the form of a soft paste obtained by forming a premix of the waxes and oils, by heating this premix to a temperature at which it melts, by then introducing the molten premix and the other constituents of the oily phase, all at once or in several portions, into a screw mixer-extruder subject to a temperature gradient ranging from 80°C to 20°C, and by kneading the mixture obtained while cooling it to ambient temperature as it is conveyed to the outlet of the mixer-extruder;
- (2) incorporation of the mixture of emulsifiers in the soft paste obtained in (1), and
- (3) incorporation, with stirring, of the mixture obtained in (2) in the aqueous phase.

25. Process according to Claim 23 or 24, characterized in that stages (2) and (3) are carried out in the screw mixer-extruder of stage (1).

26. Process according to any one of Claims 23 to 25, characterized in that the oily phase exists in the form of a soft paste having a dynamic viscosity at 25°C of between 3 and 35 Pa·s, measured with a Contraves TV rotary viscometer equipped with an "MS-r4" rotor at a frequency of 60 Hz.

## Patentansprüche

1. Zusammensetzung in Form einer Öl-in-Wasser-Emulsion, die eine Ölphase enthält, die in einer wässerigen Phase dispergiert ist, dadurch gekennzeichnet, daß sie ein Gemisch von mindestens zwei nichtionischen Emulgatoren enthält, das bei Umgebungstemperatur flüssig ist und einen HLB-Wert von 6 bis 13 aufweist, und die Ölphase ein Öl und mindestens 5 Gew.-% eines Wachses oder mehrerer Wachse, bezogen auf das Gesamtgewicht der Zusammensetzung, enthält, wobei mindestens ein Wachs unter Wachsen mit einer Temperatur des Schmelzbeginns von 50 °C oder darüber ausgewählt ist.

2. Zusammensetzung nach Anspruch 1, dadurch gekennzeichnet, daß das Gemisch von Emulgatoren
(1) mindestens einen nichtionischen Emulgator mit einem HLB-Wert von 13 oder darüber
und
(2) mindestens einen nichtionischen Emulgator mit einem HLB-Wert von 5 oder darunter
enthält.

3. Zusammensetzung nach Anspruch 1 oder 2, dadurch gekennzeichnet, daß das Gemisch von Emulgatoren ferner mindestens einen Coemulgator enthält.

4. Zusammensetzung nach Anspruch 2 oder 3, dadurch gekennzeichnet, daß der nichtionische Emulgator mit einem HLB-Wert von 13 oder darüber ausgewählt ist unter:
Estern von Polyethylenglykol mit Fettsäuren mit einer Alkylkette von 12 bis 22 Kohlenstoffatomen, die 5 bis 100 Oxyethylen-Gruppen enthalten;
Ethern von Polyethylenglykol mit Fettalkoholen mit einer Alkylkette von 12 bis 22 Kohlenstoffatomen, die 5 bis 100 Oxyethylen-Gruppen enthalten;
Estern von Sorbitan mit Fettsäuren mit einer Alkylkette von 12 bis 22 Kohlenstoffatomen, die 0 bis 100 Oxyethylen-Gruppen enthalten;
Estern von Zuckern mit Fettsäuren mit einer Alkylkette von 12 bis 22 Kohlenstoffatomen;
Derivaten von Polyethylenglykol mit Glycerinestern von Fettsäuren mit einer Alkylkette von 12 bis 22 Kohlenstoffatomen;
Ethern von Polyethylenglykol mit Estern von Methylglucose mit Fettsäuren mit einer Alkylkette von 12 bis 22 Kohlenstoffatomen, sowie Gemischen dieser Stoffe.

5. Zusammensetzung nach einem der Ansprüche 2 bis 4, dadurch gekennzeichnet, daß der nichtionische Emulgator mit einem HLB-Wert von 5 oder darunter ausgewählt ist unter
Estern von Polyolen mit Fettsäuren mit einer Alkylkette von 12 bis 22 Kohlenstoffatomen,
ethoxylierten Derivaten der Ester von Polyolen mit Fettsäuren mit einer Alkylkette von 12 bis 22 Kohlenstoffatomen, die 1 bis 50 Oxyethylen-Gruppen aufweisen,
Ethern von Polyethylenglykol mit Fettalkoholen mit einer Alkylkette von 12 bis 22 Kohlenstoffatomen, die 5 bis 100 Oxyethylen-Gruppen aufweisen, ferner
Gemischen dieser Stoffe.

6. Zusammensetzung nach einem der Ansprüche 3 bis 5, dadurch gekennzeichnet, daß der Coemulgator ausgewählt ist unter Fettalkoholen mit einer verzweigten oder ungesättigten Kette mit 8 bis 22 Kohlenstoffatomen,
Fettsäuren mit einer verzweigten oder ungesättigten Kette mit 8 bis 22 Kohlenstoffatomen,
Estern von Polyolen mit verzweigten Fettsäuren mit 8 bis 22 Kohlenstoffatomen, ferner
Gemischen dieser Stoffe.

7. Zusammensetzung nach einem der vorhergehenden Ansprüche, dadurch gekennzeichnet, daß das Gemisch von Emulgatoren Glycerylstearat, Propylenglykolstearat, Glycerylisostearat, Propylenglykoliostearat, Oleth-25 und Ceteth-25 enthält.

8. Zusammensetzung nach einem der vorhergehenden Ansprüche, dadurch gekennzeichnet, daß die wirkstoffbezogene Menge des Gemischs von Emulgatoren 0,1 bis 30 Gew.-%, bezogen auf das Gesamtgewicht der Zusammensetzung, beträgt.

9. Zusammensetzung nach einem der vorhergehenden Ansprüche, dadurch gekennzeichnet, daß das Wachs ausgewählt ist unter anorganischen Wachsen, tierischen Wachsen, pflanzlichen Wachsen, hydrierten Ölen, Fettsäureestern und Glyceriden, die bei 25 °C fest sind, synthetischen Wachsen und Siliconwachsen, ferner Gemischen dieser Stoffe.

10. Zusammensetzung nach einem der vorhergehenden Ansprüche, dadurch gekennzeichnet, daß das Wachs ausgewählt ist unter Carnaubawachs, Polyethylenwachsen mit einer Temperatur des Schmelzbeginns von über 65 °C und mikrokristallinen Wachsen mit einer Temperatur des Schmelzbeginns von über 65 °C, ferner Gemischen dieser Stoffe.

11. Zusammensetzung nach einem der vorhergehenden Ansprüche, dadurch gekennzeichnet, daß die Menge an Wachs(en) 5 bis 30 Gew.-%, bezogen auf das Gesamtgewicht der Zusammensetzung, beträgt.

12. Zusammensetzung nach einem der vorhergehenden Ansprüche, dadurch gekennzeichnet, daß die Menge der Ölphase 10 bis 70 Gew.-%, bezogen auf das Gesamtgewicht der Zusammensetzung, beträgt.

13. Zusammensetzung nach einem der vorhergehenden Ansprüche, dadurch gekennzeichnet, daß die Ölphase ferner einen oder mehrere Fettstoffe enthält, die ausgewählt sind unter Ölen tierischen Ursprungs, Ölen pflanzlichen Ursprungs, Mineralölen, synthetischen Ölen, fluorierten Ölen, Siliconölen und insbesondere flüchtigen Siliconölen, Siliconkautschuken, Siliconharzen, Fettalkoholen, Fettsäuren und Siliconelastomeren.

14. Zusammensetzung nach einem der vorhergehenden Ansprüche, dadurch gekennzeichnet, daß sie ferner mindestens einen Füllstoff enthält.

15. Zusammensetzung nach dem vorhergehenden Anspruch, dadurch gekennzeichnet, daß der Füllstoff ausgewählt ist unter Talk, Glimmern, Kaolin, Oxiden von Zink oder Titan, Calciumcarbonat, Magnesiumcarbonat und Magnesiumhydrogencarbonat, Siliciumdioxid, Titandioxid, Glaskugeln und Keramikkugeln, Metallseifen, nichtexpandierten Pulvern von synthetischen Polymeren, expandierten Pulvern, Pulvern von natürlichen organischen Materialien und Mikrokügelchen aus Siliconharz, ferner Gemischen dieser Stoffe.

16. Zusammensetzung nach Anspruch 14 oder 15, dadurch gekennzeichnet, daß die Menge an Füllstoff(en) 1 bis 12 Gew.-%, bezogen auf das Gesamtgewicht der Zusammensetzung, beträgt.

17. Zusammensetzung nach einem der vorhergehenden Ansprüche, dadurch gekennzeichnet, daß die wässerige Phase 50 bis 80 Gew.-%, bezogen auf das Gesamtgewicht der Zusammensetzung, ausmacht.

18. Zusammensetzung nach einem der vorhergehenden Ansprüche, dadurch gekennzeichnet, daß die wässerige Phase mindestens einen unter den Polyacrylamiden ausgewählten Gelbildner enthält.

19. Zusammensetzung nach einem der vorhergehenden Ansprüche, dadurch gekennzeichnet, daß sie eine kosmetische und/oder dermatologische Zusammensetzung darstellt.

20. Kosmetische, nichttherapeutische Verwendung der Zusammensetzung nach einem der Ansprüche 1 bis 18 zur Behandlung, zum Schutz, zur Pflege und/oder zur Reinigung der Haut, der Schleimhäute und/oder der Haare und/oder zum Schminken der Haut und/oder der Schleimhäute.

21. Kosmetische, nichttherapeutische Verwendung der Zusammensetzung nach einem der Ansprüche 1 bis 18 zur Behandlung von Falten und/oder Fältchen der Haut.

22. Verwendung der Zusammensetzung nach einem der Ansprüche 1 bis 18 zur Herstellung einer dermatologischen Zusammensetzung, die zur Behandlung und/oder zum Schutz von trockener Haut vorgesehen ist.

23. Verfahren zur Herstellung einer Zusammensetzung nach einem der Ansprüche 1 bis 19, dadurch gekennzeichnet, daß mindestens eine Verfahrensstufe mit einem Schnecken-Mischextruder durchgeführt wird.

24. Verfahren nach Anspruch 23, dadurch gekennzeichnet, daß es folgende Stufen umfaßt:
- (1) Herstellung der Ölphase in Form einer weichen Paste, die erhalten wird durch Vormischen der Wachse und Öle, Erhitzen dieses Vorgemisches auf eine Temperatur, bei der es schmilzt, anschließende Einführung des geschmolzenen Vorgemisches und der anderen Bestandteile der Ölphase auf einmal oder mehrmals in einen Schnecken-Mischextruder unter Anwendung eines von 80 bis 20 °C reichenden Temperaturgradienten und Kneten des erhaltenen Gemisches unter Abkühlung bis auf Umgebungstemperatur, während es zum Ausgang des Mischextruders gelangt,
- (2) Einbringen des Gemisches von Emulgatoren in die in (1) erhaltene weiche Paste und
- (3) Einbringen des in (2) erhaltenen Gemisches unter Rühren in die wässerige Phase.

25. Verfahren nach Anspruch 23 oder 24, dadurch gekennzeichnet, daß die Stufen (2) und (3) in dem Schnecken-Mischextruder von Stufe (1) durchgeführt werden.

26. Verfahren nach einem der Ansprüche 23 bis 25, dadurch gekennzeichnet, daß die Ölphase in Form einer weichen Paste mit einer dynamischen Viskosität von 3 bis 35 Pa·s bei 25 °C vorliegt, gemessen mit einem Rotationsviskosimeter CONTRAVES TV, das mit einem Einsatz "MS-r4" ausgerüstet ist, bei einer Frequenz von 60 Hz.
